# EUROPEAN PATENT SPECIFICATION

(11) **EP 1 390 490 B1**
(45) Date of publication and mention of the grant of the patent: **15.04.2009**
(21) Application number: 02739449.3
(22) Date of filing: 22.05.2002
(51) Int. Cl.: C12N 15/00, C12N 5/00, C12N 1/20, C12P 21/04, A01N 43/04, A01N 63/00

(54) **MUSCLE-SPECIFIC EXPRESSION VECTORS**
MUSKELSPEZIFISCHE EXPRESSIONSVEKTOREN
VECTEURS D'EXPRESSION SPECIFIQUES AUX MUSCLES

(30) Priority: 24.05.2001 US 293304 P
(43) Date of publication of application: 25.02.2004
(60) Divisional application: 08103273.2
(73) Proprietor: GENZYME CORPORATION, Cambridge, MA 02141 (US)
(72) Inventor: SOUZA, David, Waltham, MA 02451 (US); ARMENTANO, Donna, Belmont, MA 02478 (US)
(74) Representative: Adams, Harvey Vaughan John
(86) International application number: PCT/US2002/016748
(87) International publication number: WO 2002/095006

(56) References cited:
- US-A- 5 858 351
- US-A1- 2002 086 392
- US-B1- 6 310 196
- LI XUYANG ET AL: "Synthetic muscle promoters: Activities exceeding naturally occurring regulatory sequences" NATURE BIOTECHNOLOGY, vol. 17, no. 3, March 1999 (1999-03), pages 241-245, XP002209991 ISSN: 1087-0156
- CORDIER LAURENCE ET AL: "Muscle-specific promoters may be necessary for adeno-associated virus-mediated gene transfer in the treatment of muscular dystrophies" HUMAN GENE THERAPY, vol. 12, no. 2, 20 January 2001 (2001-01-20), pages 205-215, XP002318535 ISSN: 1043-0342
- LI S ET AL: "Muscle-specific enhancement of gene expression by incorporation of SV40 enhancer in the expression plasmid" GENE THERAPY, vol. 8, no. 6, March 2001 (2001-03), pages 494-497, XP002318536 ISSN: 0969-7128
- DATABASE EMBL [Online] 16 July 1988 (1988-07-16), "Coturnix coturnix troponin I gene, complete cds." XP002318537 retrieved from EBI accession no. EM_PRO:CCTROPIA Database accession no. CCTROPIA
- DATABASE EMBL [Online] 22 April 1989 (1989-04-22), "Mouse muscle creatine kinase (MCK) gene, 5' flank." XP002318538 retrieved from EBI accession no. EM_PRO:MMMCKA Database accession no. MMMCKA
- TRASK ET AL.: 'Developmental regulation and tissue-specific expression of the human muscle creatine kinase gene' J. BIOL. CHEM. vol. 263, no. 32, November 1988, pages 17142 - 17149, XP002958238
- LI ET AL.: 'High level desmin expression depends on a muscle-specific enhancer' J. BIOL. CHEM. vol. 266, no. 10, April 1991, pages 6562 - 6570, XP002097609
- LI ET AL.: 'Human desmin-coding gene: complete nucleotide sequence, characterization and regulation of expression during myogenesis and development' GENE vol. 78, 1989, pages 243 - 254, XP002958239
- LI ET AL: 'An E box in the desmin promoter cooperates with the E box and MEF-2 sites of a distal enhancer to direct muscle-specific transcription' EMBO J. vol. 13, no. 15, 1994, pages 3580 - 3589, XP002006941
- LI ET AL: 'Different factores interact with myoblast-specific and myotube-specific enhancer regions of the human desmin gene' J. BIOL. CHEM. vol. 268, no. 14, May 1993, pages 10403 - 10415, XP002006942

## Description

This application claims priority from U.S. provisional application No. 60/293,304 filed on May 24, 2001.

### DESCRIPTION OF THE INVENTION

### Field of the Invention

This invention relates to gene therapy methods utilizing tissue-specific expression vectors. The invention further relates to expression vectors used for delivery of a transgene into the muscle. More specifically, the invention relates to transcriptional regulatory elements that provide for enhanced and sustained expression of a transgene in the muscle.

### Background of the Invention

Gene therapy is the intracellular delivery of exogenous genetic material that corrects an existing defect or provides a new beneficial function to the cells. The muscle is an important target tissue for gene therapy because of its ready accessibility for direct injection, a relatively easy and minimally invasive method. Additionally, the muscle permits greater expression persistence compared to tissues with a higher cellular turnover rate. Skeletal muscle, for example, is being explored as a target tissue for gene therapy in a variety of therapeutic applications. There are a large number of known diseases caused by defects in gene products that could benefit from production of a protein secreted by the muscle. Familial hypercholesterolemia, hemophilia, Gaucher's and Fabry diseases, and type II diabetes are just a few examples. Many such diseases may be amenable to gene therapy (Siatskas et al., J. Inherit. Metab. Dis. 2001, 24(Suppl. 2): 25-41; Barranger et al., Expert Opin. Biol. Ther. 2001, 1(5): 857-867; Barranger et al., Neurochem. Res. 1999, 24(5): 601-615).

Various expression vectors have been developed to deliver exogenous genetic material into various tissues and organs, and muscle tissue, in particular. For a review, see Gene Expression Systems, Eds. J.M. Fernandez and J.P. Hoeffler, Academic Press, San Diego, CA, 1999. Generally, each expression system possesses certain disadvantages and obtaining desired levels of expression in vivo in a sustainable manner can be a challenge.

For example, the nucleic acid of retroviral vectors is capable of integrating into the host genome, which results in sustained expression of the transgene carried by the vector. However, the infectivity of retroviral vectors depends on on-going cell proliferation. As a consequence, in vivo delivery of these vectors can be poor. On the other hand, when adenoviral gene transfer vectors are delivered by systemic injection, high levels of transgene expression are observed (Rosefeld et al., Science 1991, 252: 431-434), but such expression can be transient and may require repeated injections. A neutralizing host immune response can further limit the effectiveness of viral vectors (Yang et al., Proc. Natl. Acad. Sci. U. S. A. 1994, 91: 4407-4411; Kozarsky et al., J. Biol. Chem. 1994, 269: 13695-13702). Non-viral gene transfer methods, such as injection of naked plasmid DNA, have also been described but the levels of gene transfer are generally too low to be sufficient for clinical applications (Malone et al., J. Biol. Chem. 1994, 269: 29903-29907; Hickman et al., Hum. Gene Ther. 1994, 5:1477-1483).

Although the muscle is highly vascularized, secretion of transgene products into the circulation can be somewhat poor. In addition to low secretion, the potentially low levels of transgene expression from muscle-specific vectors can limit the scope of gene therapy applications to those requiring low levels of circulating therapeutic proteins. Another challenge for gene therapy can be delivering the agent to a selected tissue in highly targeted manner. Effective transfection of a large and distributed tissue such as muscle usually necessitates systemic delivery. However, most known expression vectors, viral and non-viral, have potentially adverse side effects associated with ectopic expression following systemic administration. Tissue-specific expression can overcome this problem. Tissue-specific expression can be achieved through the use of transcriptional regulatory elements such as promoters and enhancers that are active only in the target tissue.

Accordingly, a primary object of the invention is to provide expression vectors optimized for sustained expression of a transgene in muscle tissue. Another object of this invention is to provide enhancer/promoter combinations that can direct sustained and appropriate expression levels in various expression systems.

These objects are achieved by combining minimal sequences from muscle-specific promoters and muscle-specific enhancers to create chimeric regulatory elements that drive transcription of a transgene in a sustained fashion. The resulting chimeric regulatory elements are useful for gene therapy directed at transgene expression in the muscle as well as other applications requiring long-term expression of exogenous proteins in transfected muscle cells such as myocytes. The various muscle-specific enhancer/promoter combinations of the invention may be useful in the context of adenoviral, adeno-associated viral (AAV), retroviral, and plasmid-based vectors for gene expression in cultured cells or in vivo.

### SUMMARY OF THE INVENTION

Chimeric regulatory elements useful for targeting transgene expression to the muscle are provided by the invention and set out in the appended claims. These chimeric regulatory elements of the invention comprise certain combinations of muscles-specific promoters and muscle-specific enhancers that are able to direct sustained transgene expression preferentially in the muscle.

The present invention is also directed to recombinant transgenes which comprise one or more operably linked tissue-specific regulatory elements of the invention. The tissue-specific regulatory elements, including muscle-specific promoter and enhancers, operably linked to a transgene drive its expression in myocytes and, in particular, in cardiomyocytes. The transgenes may be inserted in recombinant viral vectors for targeting expression of the associated coding DNA sequences in muscle. As set out in the claims, Muscle-specific promoters useful in the invention include mammalian muscle creatine kinase (MCK) promoter or mammalian desmin (DES) promoter. Alternatively, the promoter element is selected from the group consisting of mammalian MCK promoter, mammalian troponin I (TNNI2) promoter or mammalian skeletal alpha-actin (ASKA) promoter. In one particular embodiment, the promoter is a human promoter. In another embodiment, the promoter is a murine promoter. In certain embodiments, the promoter is truncated.

Tissue-specific enhancers useful in the present invention are selected from the group consisting of mammalian MCK enhancer, mammalian DES enhancer, and vertebrate troponin I IRE (TNI IRE, hereinafter referred to as FIRE) enhancer. In one embodiment, the enhancer is mammalian MCK enhancer or mammalian desmin (DE, hereinafter referred to as DES) enhancer. In another embodiment, the enhancer is mammalian DES enhancer or vertebrate FIRE enhancer. One or more of these muscle-specific enhancer elements may be used in combination with a muscle-specific promoter as disclosed herein to provide a tissue-specific regulatory element of the invention. In one embodiment, the enhancers are derived from human or mouse. In another embodiment, the FIRE enhancer is an avian enhancer. In one embodiment, the FIRE enhancer is a quail enhancer. In certain embodiments, the enhancer/enhancer or enhancer/promoter combinations are heterologous, i.e., derived from more than one species. In other embodiments, the enhancers and promoters are derived from the same species. In certain embodiments, enhancer elements are truncated.

In a particular embodiment, a regulatory element of the invention comprises at least one MCK enhancer operably linked with a DES promoter. In a related embodiment, the regulatory element additionally comprises at least one FIRE enhancer, and optionally, at least one DES enhancer. In another embodiment, a regulatory element of the invention comprises at least two MCK enhancers linked to a MCK promoter or a DES promoter. In yet another embodiment, a regulatory element comprises at least two DES enhancers linked to a DES promoter.

The invention includes vectors comprising a regulatory element of the invention. In some embodiments, the regulatory element is incorporated in non-viral plasmid-based vectors. In other exemplary embodiments, a regulatory element of the invention is incorporated into a viral vector such as one derived from adenoviruses, adeno-associated viruses (AAV), or retroviruses, including lentiviruses such as the human immunodeficiency (HIV) virus. The vectors of the invention may be used in methods of transfecting muscle tissue.

The invention further includes cells transfected with the nucleic acid containing an enhancer/promoter combination of the invention.

Additional objects and advantages of the invention will be set forth in part in the description which follows, and in part will be obvious from the description, or may be learned by practice of the invention. The objects and advantages of the invention will be realized and attained by means of the elements and combinations particularly pointed out in the appended claims.

It is to be understood that both the foregoing general description and the following detailed description are exemplary and explanatory only and are not restrictive of the invention, as claimed.

### BRIEF DESCRIPTION OF THE FIGURES

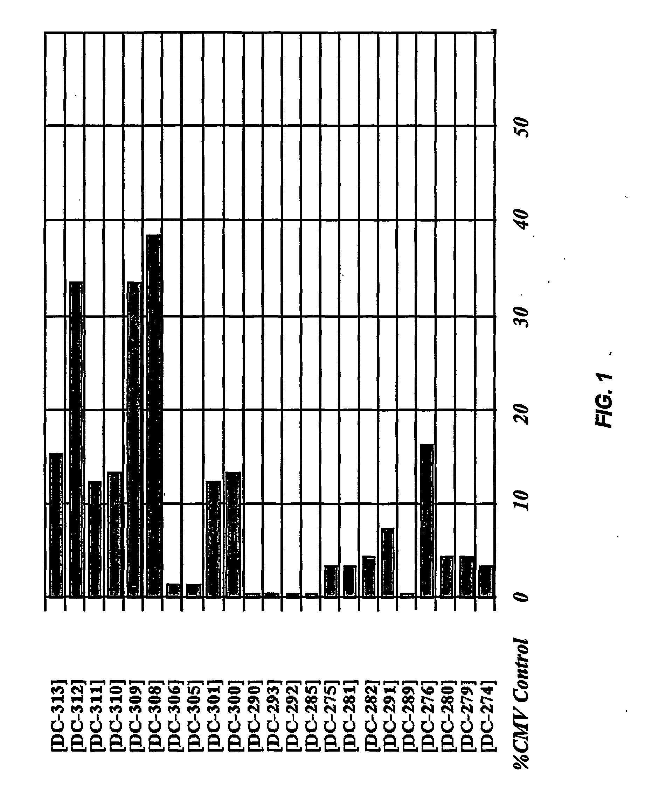

Figure 1 is a diagram representing levels of secreted alkaline phosphatase (SEAP) in the serum after intramuscular injection with test plasmids, comprising various enhancer/promoter combinations. Five mice per test plasmid were used. The amounts of serum SEAP measured at 3 days post-injection are represented as a percentage of the control group injected with a plasmid containing human cytomegalovirus (CMV) promoter and enhancer elements. Test plasmids are denoted as per Table 1.

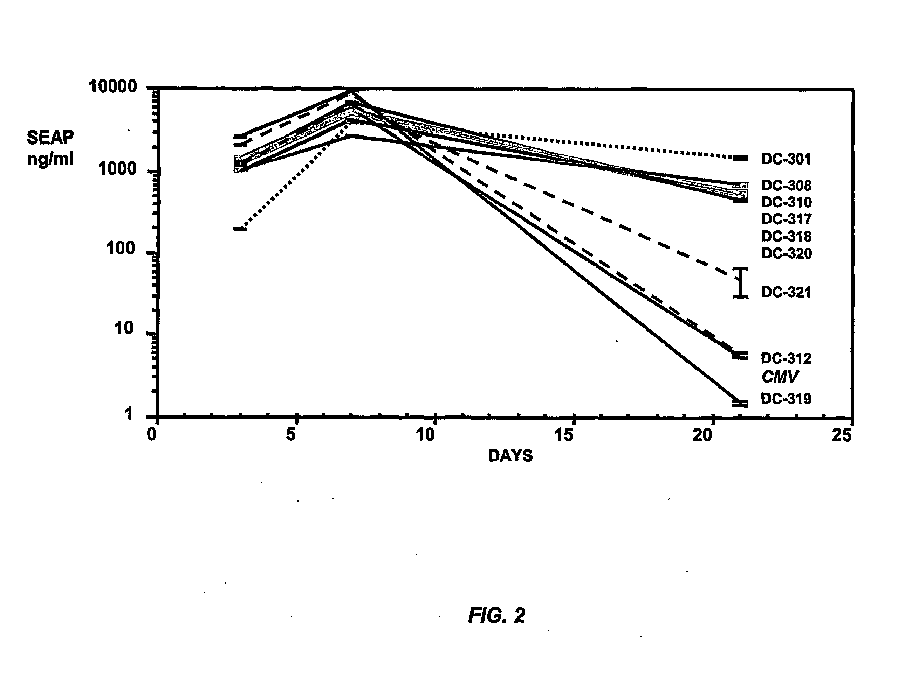

Figure 2 depicts a graph illustrating the expression levels of serum SEAP for up to 3 weeks following systemic administration of plasmids comprising various enhancer/promoter combinations. Serum SEAP levels were measured at 3, 7, and 21 days post administration. Five rats per test plasmid were used. Test plasmids are denoted as per Table 1.

### DETAILED DESCRIPTION OF THE INVENTION

The term "muscle-specific" is used, where appropriate, interchangeably with "tissue-specific" or "tissue-preferential" and refers to the capability of regulatory elements, such as promoters and enhancers, to drive expression of transgenes exclusively or preferentially in muscle tissue or muscle cells regardless of their source.

The term "myocyte," as used herein, refers a cell that has been differentiated from a progenitor myoblast such that it is capable of expressing muscle-specific phenotype under appropriate conditions. Terminally differentiated myocytes fuse with one another to form myotubes, a major constituent of muscle fibers. The term "myocyte" also refers to myocytes that are de-differentiated. The term includes cells in vivo and cells cultured ex vivo regardless of whether such cells are primary or passaged.

The term "stringent conditions" in the context of nucleic acid hybridization is intended to describe conditions of incubation and washes under which oligonucleotides that have significantly identical or homologous sequences can hybridize, i.e., complementarily bind. The conditions are selected such that sequences that are at least 20 nucleotides long and at least about 70% identical can be hybridized. Stringent conditions are well known in the art and their examples can be found in Current Protocols in Molecular Biology, Ausubel et al., eds., John Wiley & Sons, Inc. 1995, and in Sambrook et al., Molecular Cloning: A Laboratory Manual, 2nd ed. Cold Spring Harbor Press 1989. Percent identity between two sequences is determined using Basic Local Alignment Tool (BLAST) as described in Altshul et al., J. Mol. Biol. 1990, 215: 403-410.

The term "transgene" is used interchangeably with "inserted gene," or "expressed gene" and, where appropriate, "gene." "Transgene" refers to a polynucleotide that, when introduced into a cell, is capable of being transcribed under appropriate conditions so as to confer a beneficial property to the cell such as, for example, expression of a therapeutically useful protein. Where appropriate, the term "transgene" should be understood to include a combination of a coding sequence and optional non-coding regulatory sequences, such as a polyadenylation signal, a promoter, an enhancer, a repressor, etc.

The term "transfection" is used interchangeably with the terms "gene transfer," "transformation," and transduction," and means the intracellular introduction of a polynucleotide. "Transfection efficiency" refers to the relative amount of the transgene taken up by the cells subjected to transfection. In practice, transfection efficiency is estimated by the amount of the reporter gene product expressed following the transfection procedure.

The term "transfection agent," as used herein, describes substances that may facilitate the transfer of the polynucleotide across the cell wall.

The term "vector" is used interchangeably with "transgene delivery vector," "expression vector," "expression module," "expression cassette," "expression construct," and, where appropriate, "nucleic acid of the invention." "Vector" refers to viral or non-viral, prokaryotic or eukaryotic, DNA or RNA sequences that are capable of being transfected into a cell, referred to as "host cell," so that all or a part of the sequences is transcribed. It is not necessary for the transcript to be expressed. It is also not necessary for a vector to comprise a transgene having a coding sequence. Vectors are frequently assembled as composites of elements derived from different viral, bacterial, or mammalian genes. Vectors contain various coding and non-coding sequences, such as sequences coding for selectable markers, sequences that facilitate their propagation in bacteria, or one or more transcription units that are expressed only in certain cell types. For example, mammalian expression, vectors often contain both prokaryotic sequences that facilitate the propagation of the vector in bacteria and one or more eukaryotic transcription units that are expressed only in eukaryotic cells. It will be appreciated by those skilled in the art that the design of the expression vector can depend on such factors as the choice of the host cell to be transformed, the level of expression of protein desired, etc.

The term "promoter" is used interchangeably with "promoter element" and "promoter sequences." Likewise, the term "enhancer" is used interchangeably with "enhancer element" and "enhancer sequence." "Promoter" refers to a minimal sequence of a transgene that is sufficient to initiate transcription of a coding sequence of the transgene. Promoters may be constitutive or inducible. A constitutive promoter is considered to be a strong promoter if it drives expression of a transgene at a level comparable to that of the cytomegalovirus promoter (CMV) (Boshart et al., Cell 1985, 41: 521). Promoters may be coupled with other regulatory sequences/elements which, when bound to appropriate intracellular regulatory factors, enhance ("enhancers") or repress ("repressors") promoter-dependent transcription. A promoter, enhancer, or repressor, is said to be "operably linked" to a transgene when such element(s) control(s) or affect(s) transgene transcription rate or efficiency. For example, a promoter sequence located proximally to the 5' end of a transgene coding sequence is usually operably linked with the transgene. As used herein, term "regulatory elements" is used interchangeably with "regulatory sequences" and refers to promoters, enhancers, and other expression control elements, or any combination of such elements.

Promoters are positioned 5' (upstream) to the genes that they control. Many eukaryotic promoters contain two types of recognition sequences: TATA box and the upstream promoter elements. The TATA box, located 25-30 bp upstream of the transcription initiation site, is thought to be involved in directing RNA polymerase II to begin RNA synthesis as the correct site. In contrast, the upstream promoter elements determine the rate at which transcription is initiated. These elements can act regardless of their orientation, but they must be located within 100 to 200 bp upstream of the TATA box.

Enhancer elements can stimulate transcription up to 1000-fold from linked homologous or heterologous promoters. Enhancer elements often remain active even if their orientation is reversed (Li et al., J. Bio. Chem. 1990, 266: 6562-6570). Furthermore, unlike promoter elements, enhancers can be active when placed downstream from the transcription initiation site, e.g., within an intron, or even at a considerable distance from the promoter (Yutzey et al., Mol. and Cell. Bio. 1989, 9:1397-1405).

As is known in the art, some variation in this distance can be accommodated without loss of promoter function. Similarly, the positioning of regulatory elements with respect to the transgene may vary significantly without loss of function. Multiple copies of regulatory elements can act in concert. Typically, an expression vector comprises one or more enhancer sequences followed by, in the 5' to 3' direction, a promoter sequence, all operably linked to a transgene followed by a polyadenylation sequence.

Many enhancers of cellular genes work exclusively in a particular tissue or cell type. In addition, some enhancers become active only under specific conditions that are generated by the presence of an inducer such as a hormone or metal ion. Because of these differences in the specificities of cellular enhancers, the choice of promoter and enhancer elements to be incorporated into an eukaryotic expression vector is determined by the cell type(s) in which the recombinant gene is to be expressed.

Enhancer elements derived from viruses generally have a broad host range and are active in a variety of tissues. For example, the SV40 early gene enhancer is promiscuously active in many cell types derived from a variety of mammalian species, and vectors incorporating this enhancer have constitutively been widely used (Dijkema et al., EMBO J. 1985, 4: 761). Two other enhancer/promoter combinations that are active in a broad range of cells are derived from long terminal repeat (LTR) of the Rous sarcoma virus genome (Gorman et al. Proc. Natl. Sci. U. S. A. 1982, 79: 6777) and from CMV (Boshart et al., Cell 1985, 41: 521)

The regulatory elements may be heterologous with regard to each other or to a transgene, that is, they may be from different species. Furthermore, they may be from species other than the host. They also may be derived from the same species but from different genes. Alternatively, they may be derived from a single gene. Desmin Regulatory Elements

Desmin is a muscle-specific cytoskeletal protein that belongs to the family of intermediate filaments that occur at the periphery of the Z disk and may act to keep adjacent myofibrils in lateral alignment. The expression of various intermediate filaments is regulated developmentally and shows tissue specificity. Vimentin, for example, is expressed in all mesenchymal derivatives as well as in the progenitors of muscle and neural tissue; keratin, in epithelial cells; glial fibrillary acidic protein, in glial cells; and neurofilament, in neural cells. Desmin, on the other hand, is expressed exclusively in smooth skeletal muscle.

Comparison of transient expression of chloramphenicol acetyltransferase (CAT) under the control of desmin upstream regulatory sequences (between nucleotides (nt) -2255 and +75 relative to the transcriptional start site) in differentiated and undifferentiated myogenic and non-myogenic cells, reveals that the human desmin promoter region, between nt -228 and +1, is sufficient for low level expression in myotubes and myoblasts, but not fibroblasts or HeLa cells (Li et al., J. Bio. Chem. 1990, 266: 6562-6570). The same transcription start region is used in different, desmin positive organs, implying that the same promoter is active in skeletal muscle, in heart and in smooth muscle cells of different origins (van Groningen et al., Biochim. Biophys. Acta 1994, 1217: 107-109). Thus, this promoter is muscle-specific. The promoter sequence of mouse, human, hamster, and rat desmin is greatly conserved, therefore, it is expected that homologues derived from various mammalian species will have similar activity.

A human desmin (DES) promoter was obtained by cloning of the 5' flanking region from nt -2194 to +1 into pCR-Blunt II-TOPO (Invitrogen, Carlsbad, CA) using the following primers:

| | |
|---|---|
| 5' primer: | |
| 3' primer: | |

Subsequently, the desmin promoter was truncated at the Pst I site (-228). The sequence of the truncated DES promoter is provided in SEQ ID NO:19.

A 280-bp enhancer located between nt -973 and -693 of the human sequence contains several sequences homologous to other muscle-specific enhancers. Unlike other muscle-specific enhancers, the desmin (DES) enhancer can function in myoblasts as well as myotubes. The DES enhancer contains two different regions, one is active in differentiated myotubes, between nt -973 and -848, the other is active in undifferentiated myoblasts, between nt -847 and -693. Deletion of the region between nt -1738 and -693 results in a more than 20-fold decrease in expression of a linked CAT gene in differentiated muscle cells and 8-fold decrease in undifferentiated myoblasts. This 280-bp enhancer is independent of orientation, position, and distance, and can activate either the desmin promoter or heterologous promoters, such as HSV tk and human vimentin, at about 14- to 50-fold in C2.7 myotubes, and 9- to 16-fold in C2.7 myoblasts (Li et al., J. Bio. Chem. 1990, 266: 6562-6570).

The sequence of human muscle-specific 243 bp DES enhancer (-973 to -731) is provided in SEQ ID NO:21. The enhancer was amplified using the following primers:

| | |
|---|---|
| 5' primer: | GGTACTAGTC CTGCCCCCAC AGCTCCTCTC (SEQ ID NO:3) |
| 3' primer: | |

### Muscle Creatine Kinase Regulatory Elements

The muscle creatine kinase (MCK) gene is highly active in all striated muscles. Creatine kinase plays an important role in the regeneration of ATP within contractile and ion transport systems. It allows for muscle contraction when neither glycolysis nor respiration is present by transferring a phosphate group from phosphocreatine to ADP to form ATP. There are four known isoforms of creatine kinase: brain creatine kinase (CKB), muscle creatine kinase (MCK), and two mitochondrial forms (CKMi). MCK is the most abundant non-mitochondrial mRNA that is expressed in all skeletal muscle fiber types and is also highly active in cardiac muscle. The MCK gene is not expressed in myoblasts, but becomes transcriptionally activate when myoblasts commit to terminal differentiation into myocytes. MCK gene regulatory regions display striated muscle-specific activity and have been extensively characterized in vivo and in vitro. The major known regulatory regions in the MCK gene include a muscle-specific enhancer located approximately 1.1 kb 5' of the transcriptional start site in mouse and a 358-bp proximal promoter. Additional sequences that modulate MCK expression are distributed over 3.3 kb region 5' of the transcriptional start site and in the 3.3-kb first intron. Mammalian MCK regulatory elements, including human and mouse promoter and enhancer elements are described in Hauser et al., Mol. Therapy 2000, 2:16-25.

The human MCK promoter (-496 to + 37) proved difficult to amplify and, as a result, the promoter was generated in three steps. In the first step, primer pairs MCK S7-MCK S11 were used to amplify a 485 bp product. In the second step, primer pairs MCK S9-MCK S12 were used to amplify a 189 bp product. In the third step, the two products were joined with MCK S11-MCK S12 primers to amplify the 533 bp promoter.

| | |
|---|---|
| S7: | ACCCTGAACC CAGGCATGC (SEQ ID NO:5) |
| S9: | GCATGCCTGG GTTCAGGT (SEQ ID NO:6) |
| S11: | CCCTGAGTTT GAATCTC (SEQ ID NO:7) |
| S12: | AAGGGGGGCT GTCTGTA (SEQ ID NO:8) |

The sequence of the MCK promoter is provided in SEQ ID NO:18.

The muscle-specific 206 bp mouse MCK enhancer (-1256 to -1051) was amplified using the following primers:

| | |
|---|---|
| 5' primer: | GGGACTAGTC CACTACGGTC TAGGCTGCCC ATG (SEQ ID NO:9) |
| 3' primer: | |

The sequence of the MCK enhancer is provided in SEQ ID NO:20.

### Troponin I Regulatory Elements

The heterodimeric troponin complex is located on the thin filament of striated muscle and acts as a calcium-sensitive molecular switch regulating contraction. It is composed of three subunits. Troponin I (Tnl) is the inhibitory subunit of a protein complex involved in calcium mediated regulation of acto-myosin ATPase during skeletal muscle contraction. There are three known isoforms: slow skeletal muscle troponin I (TNNI1), fast skeletal muscle troponin I (TNNI2), and cardiac muscle troponin I (TNNI3). Previous studies have demonstrated that the quail Tnl gene, when introduced into mouse multipotential cell or into a determined myogenic cell line, exhibits a correct myofiber-specific expression pattern, including the appropriate timing, specificity, and transcription level. In addition, Tnl genes introduced into a transgenic mice exhibit normal developmental and tissue-specific pattern.

In quail, Tnl promoter (TNNI2) is located within nt -530 and +60. In human, the promoter sequence is located within nt -146 to +19. The sequence of the TNl2 promoter is provided in SEQ ID NO:24. The human TNI2 promoter was amplified using the following primers:

| | |
|---|---|
| 5' primer: | |
| 3' primer: | GTTGGATCCA GGCCGGCAGC GGCGAGTTGG (SEQ ID NO:26) |

An important regulatory enhancer has been identified within the intron of the quail Tnl gene (Yutzey et al., Mol. and Cell. Bio. 1989, 9: 1397-1405). This region, referred to as FIRE (fast internal regulatory element), extends from nt +643 to +781 and has been shown to contain at least four regulatory elements including E-box, a MEF-2 like sequence, a CCAC box and a CAGG sequence (Nakayama et al., Mol. Cell. Bio. 1996, 16: 2408-2417). The FIRE enhancer is position- and orientation-independent and is known to confer a muscle-specific expression pattern on a series of heterologous promoters, whereas the quail Tnl promoter region ( -530 to + 60) alone cannot elicit muscle specific expression in the absence of FIRE. Thus, tissue specificity of expression can be controlled by FIRE.

The 139 bp quail FIRE enhancer was assembled by annealing a series of synthesized oligonucleotides (Fire 1 - Fire 5) as follows:

| | |
|---|---|
| Fire 1: | |
| Fire 2: | |
| Fire 3: | |
| Fire 4: | |
| Fire 5: | |

The sequence of the avian FIRE enhancer is represented in SEQ ID NO:22.

### Skeletal Alpha-Actin Regulatory Elements

The actin multigene family is an abundant protein that polymerizes to form microfilaments which, in turn, play an important role in the maintenance of cell shape, division, and motility. There are at least six actin isoforms in vertebrates: skeletal-alpha actin (SkA) and cardiac alpha-actin are expressed in striated muscle, vascular alpha-actin and enteric gamma-actin are expressed in smooth muscle, and cytoplasmic beta- and gamma-actin are expressed in non-muscle cells. SkA is co-expressed with cardiac alpha-actin in many of the same embryonic tissues. It is up-regulated in fetal myocardium and fetal ventricle, and down-regulated during post-natal development in these tissues. In adult skeletal muscle, SkA is the dominant isoform. The coding sequences of the actin genes are highly conserved during evolution (Alonso et al., J. Mol. Evol. 1987, 194: 193-206), however, the 5' and 3' UTRs are not highly conserved between different actin isoforms therefore these regions may generally provide isotype-specific probes. Several 5' flanking regions of the SkA gene have been evolutionarily conserved, e.g., in human, mouse, rat, and chicken. There is about 73% similarity of human and rodent sequences within the 250 nt of 5' flanking region (Taylor et al., Genomics 1988, 3: 323-336).

Results of transfection experiments have demonstrated that sequences upstream of the transcription start site of the rat (Melloul et al., EMBO 1984, 3: 983-990) and chicken (Grichnick et al., Nucleic Acid Res. 1986, 14: 1683-1701) SkA genes were sufficient for both stage- and tissue-specific expression. The proximal region (-153 to -87) of the SkA gene promoter is essential for modulating the increased transcription of the gene during myogenesis in L8 cells.

The sequence of the human alpha-skeletal actin (ASKA) promoter (-481 to +34) is provided in SEQ ID NO:23. The promoter was amplified using the following primers:

| | |
|---|---|
| 5' primer: | GGTGAATTCA AGTGGGAGTT TGGGGATCTG (SEQ ID NO:16) |
| 3' primer: | ATTAGGATCC AAGCGAGGCT TCACTTGGCG (SEQ ID NO:17) |

### Chimeric Regulatory Elements

The present invention is directed to recombinant transgenes which comprise one or more of the tissue-specific regulatory elements described above. The chimeric tissue-specific regulatory elements of the invention drive transgene expression in myocytes, and, in particular, in cardiomyocytes. The transgenes may be inserted in recombinant viral or non-viral vectors for targeting expression of the associated coding DNA sequences in muscle. In one embodiment, the promoter element is selected from the group consisting of mammalian muscle creatine kinase (MCK) promoter and mammalian (DES) desmin promoter. Alternatively, the promoter element is selected from the group .consisting of mammalian MCK promoter, mammalian troponin I (TNNI2) promoter, and mammalian skeletal alpha-actin (ASKA) promoter. In one particular embodiment, the promoter is a human promoter. In another embodiment, the promoter is a murine promoter. In certain embodiments, the promoter is truncated.

The tissue-specific regulatory elements of this invention include at least one enhancer selected from the group consisting of mammalian MCK enhancer, mammalian DES (also known as DE) enhancer, and vertebrate troponin I IRE (FIRE, also known as TNI IRE) enhancer. One or more of these muscle-specfic enhancer elements may be used in combination with a promoter element as disclosed herein. In one embodiment, enhancers are derived from human or mouse. In another embodiment FIRE enhancer is an avian enhancer. In a particular embodiment, the FIRE enhancer is a quail enhancer. In certain embodiments, the enhancer/enhancer or enhancer/promoter combinations are heterologous, i.e., the elements derived from more that one species. In others, they are derived from the same species. In certain embodiments, enhancer elements are truncated so that binding sites for known transcriptional repressors have been deleted.

In a particular embodiment, a regulatory element of the invention comprises at least one MCK enhancer operably linked with a DES promoter. In another embodiment, the regulatory element additionally comprises at least one FIRE enhancer, and optionally, at least one DES enhancer. In another embodiment, the regulatory element comprises at least two MCK enhancers linked to a MCK promoter or a DES promoter. In yet another embodiment, a regulatory element comprises at least two DES enhancers linked to a DES promoter.

It will be understood that the regulatory elements of the invention are not limited to specific sequences referred to in the specification but also encompass their structural and functional analogs/homologues. Such analogs may contain truncations, deletions, insertions, as well as substitutions of one or more nucleotides introduced either by directed or by random mutagenesis. Truncations may be introduced to delete one or more binding sites for known transcriptional repressors. Additionally, such sequences may be derived from sequences naturally found in nature that exhibit a high degree of identity to the sequences in the invention. A nucleic acid of 20 nt or more will be considered to have high degree of identity to a promoter/enhancer sequence of the invention if it hybridizes to such promoter/enhancer sequence under stringent conditions. Alternatively, a nucleic acid will be considered to have a high degree of identity to a promoter/enhancer sequence of the invention if it comprises a contiguous sequence of at least 20 nt, which has percent identity of at least 60%, 65%, 70%, 75%, 80%, 85%, 90%, 95%, or more as determined by standard alignment algorithms such as, for example, Basic Local Alignment Tool (BLAST) described in Altshul et al., J. Mol. Biol. 1990, 215: 403-410, the algorithm of Needleman et al., J. Mol. Biol. 1970, 48: 444-453, or the algorithm of Meyers et al., Comput. Appl. Biosci. 1988, 4: 11-17. Non-limiting examples of analogs, e.g., homologous promoters sequences and homologous enhancer sequences derived from various species, are described in the present specification.

The invention further includes vectors, comprising a regulatory element of the invention. In general, there are no known limitations on the use of the regulatory elements of the invention in any vector. In some embodiments, the regulatory elements are incorporated in non-viral plasmid-based vectors. In other exemplary embodiments, a regulatory element of the invention is incorporated into a viral vector such as derived from adenoviruses, adeno-associated viruses (AAV), or retroviruses, including lentivirus such as the human immunodeficiency (HIV) virus.

In the present invention, the transgene may comprise a DNA sequence encoding proteins involved in metabolic diseases, or disorders and diseases of muscle system, muscle wasting, or muscle repair. Vectors of the invention may include a transgene containing a sequence coding for a therapeutic polypeptide. For gene therapy, such a transgene is selected based upon a desired therapeutic outcome. It may encode, for example, antibodies, hormones, enzymes, receptors, or other proteins of interest or their fragments, such as, for example, TGF-beta receptor, glucagon-like peptide 1, dystrophin, leptin, insulin, pre-proinsulin, follistatin, PTH, FSH, IGF, EGF, TGF-beta, bone morphogeneteic proteins, other tissue growth and regulatory factors, growth hormones, and blood coagulation factors. For example, in treatment of lysosomal storage disease, one may employ transgenes coding for enzymes such as glucocerebrosidase, alpha-galactosidase, beta-glucuronidase, alpha-Liduronidase, iduronate sulphatase, alpha-N-acetylgalactosaminidase, sphingomyelinase and alpha-glucosidase. In the treatment of familial hypercholesterolemia, for example, one may use a transgene encoding LDL receptor (Kobayashi et al., J. Biol. Chem. 271: 6852-6860).

Also disclosed herein are methods of transfecting the muscle tissue where such methods utilize the vectors of the invention. It will be understood that vectors of the invention are not limited by the type of the transfection agent in which to be administered to a subject or by the method of administration. Transfection agents may contain compounds that reduce the electrostatic charge of the cell surface and the polynucleotide itself, or increase the permeability of the cell wall. Examples include cationic liposomes, calcium phosphate, polylysine, vascular endothelial growth factor (VEGF), etc. Hypertonic solutions containing, for example, NaCl, sugars, or polyols, can also be used to increase the extracellular osmotic pressure thereby increasing transfection efficiency. Transfection agent may also include enzymes such as proteases and lipases, mild detergents and other compounds that increase permeability of cell membranes. The methods disclosed herein are not limited to any particular composition of the transfection agent and can be practiced with any suitable agent so long as it is not toxic to the subject or its toxicity is within acceptable limits. Non-limiting examples of suitable transfection agents are given in this specification.

The invention also includes cells transfected with the DNA containing an enhancer/promoter combination of the invention. Standard methods for transfecting cell with isolated nucleic acid are well known to those skilled in art. Transfected cells may be used, for example, to confirm the identity of a transgene; to study biosynthesis and intracellular transport of proteins encoded by transgenes; or to culture cells ex vivo for subsequent re-implantation into a subject, etc. Methods for in vivo intramuscular injection and transfection of myocytes ex vivo are known in the art. For example, see Shah et al., Transplantation 1999, 31: 641-642; Daly et al., Human Gene Therapy 1999, 10: 85-94.

Host cells that can be used with the vectors of invention are myocytes found in all muscle types, e.g., skeletal muscle, cardiac muscle, smooth muscle, etc. Myocytes are found and can be isolated from any vertebrate species, including, without limitation, human, orangutan, monkey, chimpanzee, dog, cat, rat, rabbit, mouse, horse, cow, pig, elephant, etc. Alternatively, the host cell can be a prokaryotic cell, e.g., a bacterial cell such as E. coli, that is used, for example, to propagate the vectors.

It may be desirable in certain circumstances to utilize myocyte progenitor cells such as mesenchymal precursor cells or myoblasts rather than fully differentiated myoblasts. Examples of tissue from which such cells can be isolated include placenta, umbilical cord, bone marrow, skin, muscle, periosteum, or perichondrium. Myocytes can be derived from such cells, for example, by inducing their differentiation in tissue culture. The present invention encompasses not only myocyte precursor/progenitor cells, but also cells that can be trans-differentiated into myocytes, e.g., adipocytes and fibroblasts.

Other embodiments of the invention will be apparent to those skilled in the art from consideration of the specification and practice of the invention disclosed herein. It is intended that the specification and examples be considered as exemplary only, with a true scope and spirit of the invention being indicated by the following claims. While the representative experiments are performed in test animals, similar results are expected in humans. The invention therefore provides for the use of a vector of the invention for the preparation of a medicament for expressing a gene in the muscle. The exact parameters to be used for injections of such medicaments into humans can be easily determined by a person skilled in the art.

### EXAMPLES

### Example 1: Vector Construction

Restriction enzymes, T4 DNA ligase, DNA polymerase I and large fragment (Klenow) were purchased from New England BioLabs (Beverly, MA).

Mouse and human genomic DNA was obtained from Clontech (Palo Alto, CA). PCR-amplification of regulatory elements from genomic DNA was performed with Vent_{R}® DNA polymerase (New England BioLabs, Beverly, MA) using primers as indicated in the Detailed Description of Invention as follows: 1cycle of 4 min at 94°C, 2 min at 45°C, and 5 min at 68°C with 34 cycles of 1 min at 94°C, 2 min at 55°C, and 5 min at 68°C. SV72 enhancer element containing a 72-bp repeat from the simian virus 40 (SV40) enhancer is described in Li et al., Gene Therapy 2001, 8: 494-497. DNA restriction fragments to be cloned into phagemid or plasmid vectors were isolated from agarose gels using DEAE paper and cloned as described in Sambrook et al., Molecular Cloning: A Laboratory Manual, 2nd Ed., Cold Spring Harbor Laboratory Press, 1989.

Multiple copies of various enhancers were cloned between the Spe I and BsiW I sites in Litmus 28™ (New England BioLabs, Beverly, MA) using standard recombinant DNA methods. Human alpha skeletal actin promoter (ASKA; -481 to +34) and human troponin promoter (TNN12; -146 to +19) were cloned between the EcoR I and BamH I site in Litmus 28™ while the human muscle creatine kinase promoter (MCK; -496 to +37) and human desmin promoter (DES; -2194 to +1) were cloned into pCR- Blunt II-TOPO (Invitrogen, Carlsbad, CA). The desmin promoter was truncated at the Pst I (-228) site before adding enhancer combinations. Promoter and enhancer elements were sequenced to verify integrity.

Standard cloning techniques were used to introduce the muscle-specific promoter chimeras into the pCFA-HI-SEAP expression vector to generate muscle chimeras which direct transcription of the secreted alkaline phosphatase (SEAP) reporter gene. pCFA-HI-SEAP plasmid, also known as pCF1-SEAP, is described in Yew et al., Hum. Gen. Ther. 1997, 8: 575-584.

All plasmids were prepared using the Qiagen plasmid Maxi kit (Qiagen, Valencia, CA). Before injection into animals, the plasmids were extracted with Triton X-100® (Sigma-Aldrich, St. Louis, MO) to remove endotoxins.

The list of created plasmids is shown in Table 1, wherein "L28" stands for Litmus 28™; "HI" stands for hybrid intron (the sequence is described in MacGregor et al., Nucleic Acids Research 1989, 17: 2365); ">" stands for direct (5'→ 3') orientation of an enhancer sequence; "<" stands for inverse (3'→5') orientation of an enhancer sequence; and the number in parentheses stands for the number of enhancer elements inserted, or a nucleotide position of the promoter in the original gene, as appropriate.

### Example 2: Short-Term Expression in Mice

BALB/C mice were injected with 50 µg test plasmid in 50 µl of phosphate-buffered saline (PBS) into the anterior tibialis. Five mice were used for each test plasmid or the control group. A plasmid containing a CMV promoter/enhancer (-1 to -522) as described in Li et al., Gene Therapy 2001, 8: 494-497, was used in the control animals.

The overall efficiency of transfection was evaluated by measuring the concentration of SEAP in the serum of animals. Blood was collected intraorbitally at 7 days post-injection. The serum was heated to 65°C to denature endogenous alkaline phosphatase and assayed for SEAP activity per manufacturer's instructions using an alkaline phosphatase reagent from Sigma-Aldrich (St. Louis, MO) and human placental alkaline phosphatase from Calbiochem (LaJolla, CA) as a standard. The observed SEAP expression levels were normalized as a percentage of the CMV control.

SEAP expression levels of various promoter/enhancer chimeras, calculated as a mean of each group, are presented in Figure 1. As is demonstrated in Figure 1, truncated desmin promoter constructs DC-308, DC-309, DC-310, and DC-312 exhibited expression levels greater than one third of the CMV control. ASKA chimera DC-276 and MCK chimeras DC-300 and DC-301 expressed SEAP at about half the expression levels of the desmin promoter constructs, while others expressed at below than 10% of the CMV control.

### Example 3: Long-Term Expression in Rats

To investigate persistence of expression in various enhancer/promoter combinations, Sprague Dawley rats were injected into iliac vein with 500 µg test plasmid in 500 µl of phosphate-buffered saline (PBS). Five rats were used for each test plasmid or a control group. A plasmid containing a CMV promoter/enhancer (-1 to -522) as described in Li et al., Gene Therapy 2001, 8: 494-497 was used in control animals. Blood was collected at 1, 7, and 21 days post-injection, and the serum was assayed for SEAP activity as described in Example 1.

Comparisons of SEAP expression levels among various promoter/enhancer chimeras were made. The SEAP expression levels, calculated as a mean of each group, are presented in Figure 2 and, in a tabulated form, in Table 2. As is demonstrated, comparable SEAP expression levels to the CMV control were achieved by day 7 in all chimeras tested but, by day 21, DC-301 demonstrated the greatest expression persistence. With DC-308, DC-310, and DC-312, SEAP expression levels were slightly better in rats than in mice. The desmin or MCK enhancers linked to either the desmin or MCK promoters yielded good persistent expression. This is exemplified in Figure 2 with the following constructs: DC-301, DC-308, DC-310, DC-317, DC-318, and DC-320.

The specification is most thoroughly understood in light of the teachings of the references cited within the specification. The embodiments within the specification provide an illustration of embodiments of the invention and should not be construed to limit the scope of the invention. The skilled artisan recognizes that many other embodiments are encompassed by the claimed invention and that it is intended that the specification and examples be considered as exemplary only, with a true scope and spirit of the invention being indicated by the following claims.

**Table 1 List of Test Plasmids**

| **Designation of Construct** | **Features** |
|---|---|
| DC-274 | L28 ASKA HI SEAP |
| DC-275 | L28 FIRE(2) TNNI2 HI SEAP |
| DC-276 | L28 MCK(2) ASKA HI SEAP |
| DC-279 | L28 FIRE ASKA HI SEAP |
| DC-280 | L28 FIRE(2) ASKA HI SEAP |
| DC-281 | L28 TNNI2 HI SEAP |
| DC-282 | L28 DES ASKA HI SEAP |
| DC-285 | MCK(-496 to +37) HI SEAP |
| DC-289 | L28 MCK(4) ASKA HI SEAP |
| DC-290 | MCK(-496 to +37) HI FIRE(2) SEAP |
| DC-291 | L28 MCK(2) DES ASKA HI SEAP |
| DC-292 | >DES(1) MCK(-496 to +37) HI SEAP |
| DC-293 | <DES(1) MCK(-496 to +37) HI SEAP |
| DC-300 | >MCK(2) MCK(-496 to +37) HI SEAP |
| DC-301 | <MCK(2) MCK(-496 to +37) HI SEAP |
| DC-305 | DES(-228) HI SEAP |
| DC-306 | DES(-2194) HI SEAP |
| DC-308 | DES(2) DES(-228) HI SEAP |
| DC-309 | DES(4) DES(-228) HI SEAP |
| DC-310 | MCK(2) DES(-228) HI SEAP |
| DC-311 | MCK(4) DES(-228) HI SEAP |
| DC-312 | FIRE(2) DES(-228) HI SEAP |
| DC-313 | FIRE(4) DES(-228) |
| DC-317 | MCK(2) FIRE(2) DES(-228) HI SEAP |
| DC-318 | DES(2) MCK(2) DES(-228) HI SEAP |
| DC-319 | FIRE(2) DES(2) DES(-228) HI SEAP |
| DC-320 | DES(2) SV72 DES(-228) HI SEAP |
| DC-321 | FIRE(2) SV72 DES(-228) HI SEAP |

**Table 2 SEAP Expression (ng/ml) in rats as a function of time**

| Days post-injection | **3** | **7** | **21** |
|---|---|---|---|
| **Construct** | | | |
| **DC-301** | 194.66 | 3944.17 | 1512.65 |
| **DC-308** | 1257.28 | 5070.39 | 491.83 |
| **DC-310** | 1053.40 | 4157.77 | 570.86 |
| **DC-312** | 1497.57 | 5803.4 | 5.42 |
| **DC-317** | 6864.08 | 6864.08 | 458.64 |
| **DC-318** | 1473.79 | 5538.83 | 566.39 |
| **DC-319** | 2679.61 | 9123.79 | 1.47 |
| **DC-320** | 1057.28 | 2694.17 | 717.59 |
| **DC-321** | 1215.53 | 6264.56 | 48.0 |
| **CMV HI SEAP** | 2133.98 | 8628.64 | 5.8 |

## Claims

1. A recombinant muscle-specific regulatory element for gene expression, comprising:
a) a promoter element selected form the group consisting of the mammalian muscle creatine kinase (MCK) promoter and mammalian desmin (DES) promoter, and
b) at least one vertebrate troponin I IRE (FIRE) enhancer, or at least two mammalian muscle creatine kinase (MCK) enhancers
wherein the promoter and enhancer elements are operably linked.

2. The regulatory element of claim 1 comprising at least two mammalian muscle creatine kinase (MCK) enhancers and a mammalian muscle creatine kinase (MCK) promoter, or a mammalian desmin (DES) promoter.

3. A recombinant muscle-specific regulatory element for gene expression, comprising:
a) a mammalian desmin (DES) promoter; and
b) at least one mammalian muscle creatine kinase (MCK) enhancer, or at least one vertebrate troponin I IRE (FIRE) enhancer, or at least two mammalian desmin (DES) enhancers, or a combination thereof
wherein the promoter and enhancer elements are operably linked.

4. The regulatory element of claim 3 comprising at least one mammalian muscle creatine kinase (MCK) enhancer and a mammalian desmin (DES) promoter.

5. The regulatory element of claim 3 comprising at least one mammalian muscle creatine kinase (MCK) enhancer, at least one vertebrate troponin I IRE (FIRE) enhancer and a mammalian desmin (DES) promoter.

6. The regulatory element of claim 5 further comprising at least one mammalian desmin (DES) enhancer.

7. The regulatory element of claim 3 comprising at least two mammalian desmin (DES) enhancers and a mammalian desmin (DES) promoter.

8. The regulatory element of claim 7 further comprising at least two mammalian muscle creatine kinase (MCK) enhancers or at least two vertebrate troponin I IRE (FIRE) enhancers.

9. A recombinant muscle-specific regulatory element for gene expression, comprising at least one mammalian desmin (DES) enhancer and a mammalian muscle creatine kinase (MCK) promoter, wherein the promoter and enhancer elements are operably linked.

10. The regulatory element of any of the claims 1-9, wherein the promoter element is a human promoter, or murine.

11. The regulatory element of any of the claims 1, 2, or 9, wherein the MCK promoter comprises the human MCK promoter of SEQ ID NO:18.

12. The regulatory element of any of the claims 1-8, wherein the DES promoter comprises the human DES promoter of SEQ ID NO:19.

13. The regulatory element of any of the claims 1-9, wherein the enhancer element is a human enhancer, or a murine enhancer.

14. The regulatory element of any of the claims 1-6, or 8, wherein the regulatory element comprises the mouse MCK enhancer of SEQ ID NO:20.

15. The regulatory element of any of the claims 3 or 6-9, wherein the regulatory element comprises the human DES enhancer of SEQ ID NO:21.

16. The regulatory element of any of the claims 1, 3, 5, 6, or 8, wherein the regulatory element comprises a FIRE enhancer which is an avian enhancer, a mammalian enhancer, or a human enhancer.

17. The regulatory element of claim 16 wherein said FIRE enhancer comprises the quail troponin I enhancer of SEQ ID NO:22.

18. The regulatory element of claims 1-7, 8, or 9, wherein the promoter and enhancer elements are derived from the same species, or wherein the promoter and enhancer elements are derived from different species.

19. A vector comprising the recombinant muscle-specific regulatory element for gene expression that is defined by any one of claims 1-18.

20. The vector as in claim 19, wherein the vector is selected from the group consisting of a plasmid and a viral vector.

21. The vector as in claim 20, wherein the viral vector is derived from a virus selected from the group consisting of an adenovirus, an adeno-associated virus, and a retrovirus.

22. The vector as in claim 21, wherein the retrovirus is a lentivirus.

23. Use of the vector according to claim 19 for the preparation of a medicament for expressing a gene in the muscle.

24. A transfected host cell comprising the vector according to claim 19.

25. The transfected host cell of claim 24, wherein the host cell is a prokaryotic cell, or a mammalian cell.

26. The transfected host cell of claim 24, wherein the host cell is a myocyte.

27. A recombinant transgene useful for expression of a coding sequence, comprising a strong constitutive promoter and one or more muscles-specific enhancer elements, wherein the strong constitutive promoter is mammalian skeletal alpha-actin (ASKA) promoter and the muscle-specific enhancer is selected from the group consisting of mammalian troponin I internal regulatory elements (FIRE), mammalian muscle creatine kinase (MCK) enhancers, and mammalian desmin (DES) enhancers.

28. A recombinant transgene useful for expression of a coding sequence, comprising a strong constitutive promoter and one or more muscles-specific enhancer elements, wherein the strong constitutive promoter is mammalian troponin I (TNNI2) promoter and the muscle-specific enhancer is selected from the group consisting of mammalian muscle creatine kinase (MCK) enhancers, and mammalian desmin (DES) enhancers.

29. The recombinant transgene of claim 27 or 28, wherein the promoter element is a human promoter, or murine.

30. The recombinant transgene according to claim 27 or 28, wherein the promoter is a truncated promoter from which one or more binding sites for known transcriptional repressors have been deleted.

31. A vector comprising the transgene of claim 27 or 28.

32. The vector as in claim 31, wherein the vector is selected from the group consisting of a plasmid and a viral vector.

33. The vector as in claim 32, wherein the viral vector is derived from a virus selected from an adenovirus, and adeno-associated virus, and a retrovirus.

34. The vector as in claim 33, wherein the retrovirus is a lentivirus.

35. Use of the vector according to claim 31 for the preparation of a medicament for expressing a gene in the muscle.

36. A transfected host cell comprising the vector according to claim 31.

37. The transfected host cell of claim 36, wherein the host cell is a prokaryotic cell, or a mammalian cell.

38. The transfected host cell of claim 36,wherein the host cell is a myocyte.

## Patentansprüche

1. Rekombinantes, muskelspezifisches, regulatorisches Element für die Genexpression, umfassend:
(a) ein Promotorelement, das aus der Gruppe ausgewählt ist, die besteht aus dem Säugetiermuskel-Creatin-kinase (MCK)-Promotor und dem Säugetier-Desmin (DES)-Promotor; und
(b) mindestens einen Wirbeltier-Troponin I-IRE (FIRE)-Enhancer oder mindestens zwei Säugetiermuskel-Creatin-kinase (MCK)-Enhancer,
wobei die Promotor- und Enhancerelemente funktionell verknüpft sind.

2. Regulatorisches Element nach Anspruch 1, umfassend mindestens zwei Säugetiermuskel-Creatin-kinase (MCK)-Enhancer und einen Säugetiermuskel-Creatin-kinase (MCK)-Promotor oder einen Säugetier-Desmin (DES)-Promotor.

3. Rekombinantes, muskelspezifisches, regulatorisches Element für die Genexpression, umfassend:
(a) einen Säugetier-Desmin (DES)-Promotor; und
(b) mindestens einen Säugetiermuskel-Creatin-kinase (MCK)-Enhancer oder mindestens einen Wirbeltier-Troponin I-IRE (FIRE)-Enhancer oder mindestens zwei Säugetier-Desmin (DES)-Enhancer oder eine Kombination davon,
wobei die Promotor- und Enhancerelemente funktionell verknüpft sind.

4. Regulatorisches Element nach Anspruch 3, umfassend mindestens einen Säugetiermuskel-Creatin-kinase (MCK)-Enhancer und einen Säugetier-Desmin (DES)-Promotor.

5. Regulatorisches Element nach Anspruch 3, umfassend mindestens einen Säugetiermuskel-Creatin-kinase (MCK)-Enhancer, mindestens einen Wirbeltier-Troponin I-IRE (FIRE)-Enhancer und einen Säugetier-Desmin (DES)-Promotor.

6. Regulatorisches Element nach Anspruch 5, ferner umfassend mindestens einen Säugetier-Desmin (DES)-Enhancer.

7. Regulatorisches Element nach Anspruch 3, umfassend mindestens zwei Säugetier-Desmin (DES)-Enhancer und einen Säugetier-Desmin (DES)-Promotor.

8. Regulatorisches Element nach Anspruch 7, ferner umfassend mindestens zwei Säugetiermuskel-Creatin-kinase (MCK)-Enhancer oder mindestens zwei Wirbeltier-Troponin I-IRE (FIRE)-Enhancer.

9. Rekombinantes, muskelspezifisches, regulatorisches Element für die Genexpression, umfassend mindestens einen Säugetier-Desmin (DES)-Enhancer und einen Säugetiermuskel-Creatin-kinase (MCK)-Promotor, wobei die Promotor- und Enhancerelemente funktionell verknüpft sind.

10. Regulatorisches Element nach einem der Ansprüche 1 bis 9, wobei es sich beim Promotorelement um einen humanen Promotor oder um einen murinen Promotor handelt.

11. Regulatorisches Element nach einem der Ansprüche 1, 2 oder 9, wobei der MCK-Promotor den humanen MCK-Promotor von SEQ ID NO: 18 umfasst.

12. Regulatorisches Element nach einem der Ansprüche 1 bis 8, wobei der DES-Promotor den humanen DES-Promotor von SEQ ID NO: 19 umfasst.

13. Regulatorisches Element nach einem der Ansprüche 1 bis 9, wobei es sich beim Enhancerelement um einen humanen Enhancer oder um einen murinen Enhancer handelt.

14. Regulatorisches Element nach einem der Ansprüche 1 bis 6 oder 8, wobei das regulatorische Element den murinen MCK-Enhancer von SEQ ID NO: 20 umfasst.

15. Regulatorisches Element nach einem der Ansprüche 3 oder 6 bis 9, wobei das regulatorische Element den humanen DES-Enhancer von SEQ ID NO: 21 umfasst.

16. Regulatorisches Element nach einem der Ansprüche 1, 3, 5, 6 oder 8, wobei das regulatorische Element einen FIRE-Enhancer umfasst, bei dem es sich um einen avianen Enhancer, einen Säugetier-Enhancer oder einen humanen Enhancer handelt.

17. Regulatorisches Element nach Anspruch 16, wobei der FIRE-Enhancer den Wachtel-Troponin I-Enhancer von SEQ ID NO: 22 umfasst.

18. Regulatorisches Element nach den Ansprüchen 1 bis 7, 8 oder 9, wobei die Promotor- und Enhancerelemente sich von der gleichen Spezies ableiten oder wobei die Promotor- und Enhancerelemente sich von verschiedenen Spezies ableiten.

19. Vektor, umfassend das rekombinante, muskelspezifische, regulatorische Element für die Genexpression, das in einem der Ansprüche 1 bis 18 definiert ist.

20. Vektor nach Anspruch 19, wobei der Vektor aus der Gruppe ausgewählt ist, die besteht aus einem Plasmidvektor und einem viralen Vektor.

21. Vektor nach Anspruch 20, wobei der virale Vektor sich von einem Virus ableitet, das aus der Gruppe ausgewählt ist, die besteht aus einem Adenovirus, einem adenoassoziierten Virus und einem Retrovirus.

22. Vektor nach Anspruch 21, wobei es sich beim Retrovirus um ein Lentivirus handelt.

23. Verwendung des Vektors nach Anspruch 19 zur Herstellung eines Arzneimittels zur Expression eines Gens im Muskel.

24. Transfizierte Wirtszelle, umfassend den Vektor nach Anspruch 19.

25. Transfizierte Wirtszelle nach Anspruch 24, wobei es sich bei der Wirtszelle um eine prokaryontische Zelle oder um eine Säugetierzelle handelt.

26. Transfizierte Wirtszelle nach Anspruch 24, wobei es sich bei der Wirtszelle um einen Myozyten handelt.

27. Rekombinantes Transgen, das sich für die Expression einer Kodierungssequenz eignet, umfassend einen starken, konstitutiven Promotor und ein oder mehr muskelspezifische Enhancerelemente, wobei es sich beim starken, konstitutiven Promotor um den Säugetierskelett-alpha-Actin (ASKA)-Promotor handelt und der muskelspezifische Enhancer aus der Gruppe ausgewählt ist, die besteht aus internen, regulatorischen Säugetier-Troponin I- Elementen (FIRE), Säugetiermuskel-Creatin-kinase (MCK)-Enhancern und Säugetier-Desmin (DES)-Enhancern.

28. Rekombinantes Transgen, das sich für die Expression einer Kodierungssequenz eignet, umfassend einen starken, konstitutiven Promotor und ein oder mehr muskelspezifische Enhancerelemente, wobei es sich beim starken, konstitutiven Promotor um den Säugetier-Troponin I (TNNI2)-Promotor handelt und der muskelspezifische Enhancer aus der Gruppe ausgewählt ist, die besteht aus Säugetiermuskel-Creatin-kinase (MCK)-Enhancern und Säugetier-Desmin (DES)-Enhancern.

29. Rekombinantes Transgen nach Anspruch 27 oder 28, wobei es sich beim Promotorelement um einen humanen Promotor oder einen murinen Promotor handelt.

30. Rekombinantes Transgen nach Anspruch 27 oder 28, wobei es sich beim Promotor um einen geschnittenen Promotor handelt, aus dem ein oder mehr Bindungsstellen für bekannte transkriptionelle Repressoren deletiert worden sind.

31. Vektor, umfassend das Transgen nach Anspruch 27 oder 28.

32. Vektor nach Anspruch 31, wobei der Vektor aus der Gruppe ausgewählt ist, die besteht aus einem Plasmidvektor und einem viralen Vektor.

33. Vektor nach Anspruch 32, wobei der virale Vektor von einem Virus abgeleitet ist, das aus einem Adenovirus, einem adenoassoziierten Virus und einem Retrovirus ausgewählt ist.

34. Vektor nach Anspruch 33, wobei es sich beim Retrovirus um ein Lentivirus handelt.

35. Verwendung des Vektors nach Anspruch 31 zur Herstellung eines Arzneimittels zur Expression eines Gens im Muskel.

36. Transfizierte Wirtszelle, umfassend den Vektor nach Anspruch 31.

37. Transfizierte Wirtszelle nach Anspruch 36, wobei es sich bei der Wirtszelle um eine prokaryontische Zelle oder um eine Säugetierzelle handelt. 38. Transfizierte Wirtszelle nach Anspruch 36, wobei es sich bei der Wirtszelle um einen Myozyten handelt.

## Revendications

1. Elément de régulation spécifique aux muscles recombiné pour l'expression génique comprenant :
a) un élément promoteur choisi dans le groupe formé par le promoteur de la créatine kinase musculaire (MCK) chez le mammifère et le promoteur de la desmine (DES) chez le mammifère, et
b) au moins un élément d'amplification de troponine 1 IRE (FIRE) chez le vertébré ou au moins deux éléments d'amplification de la créatine kinase musculaire (MCK) chez le mammifère,
étant précisé que les éléments promoteurs et les éléments d'amplification sont opérationnellement liés.

2. Elément de régulation selon la revendication 1, comprenant au moins deux éléments d'amplification de la créatine kinase musculaire (MCK) chez le mammifère et un promoteur de la créatine kinase musculaire (MCK) chez le mammifère, ou un promoteur de la desmine (DES) chez le mammifère.

3. Elément de régulation spécifique aux muscles recombiné pour l'expression génique comprenant :
a) un promoteur de la desmine (DES) chez le mammifère, et
b) au moins un élément d'amplification de la créatine kinase musculaire (MCK) chez le mammifère ou au moins un élément d'amplification de la troponine 1 IRE (FIRE) chez le vertébré ou au moins deux éléments d'amplification de la desmine (DES) chez le mammifère ou une combinaison de ces éléments,
étant précisé que les éléments promoteurs et les éléments d'amplification sont opérationnellement liés.

4. Elément de régulation selon la revendication 3, comprenant au moins un élément d'amplification de la créatine kinase musculaire (MCK) chez le mammifère et un promoteur de la desmine (DES) chez le mammifère.

5. Elément de régulation selon la revendication 3, comprenant au moins un élément d'amplification de la créatine kinase musculaire (MCK) chez le mammifère et au moins un élément d'amplification de la troponine 1 IRE (FIRE) chez le vertébré et un promoteur de la desmine (DES) chez le mammifère.

6. Elément de régulation selon la revendication 5, comprenant en outre au moins un élément d'amplification de la desmine (DES) chez le mammifère.

7. Elément de régulation selon la revendication 3, comprenant au moins deux éléments d'amplification de la desmine (DES) chez le mammifère et un promoteur de la desmine (DES) chez le mammifère.

8. Elément de régulation selon la revendication 7, comprenant en outre au moins deux éléments d'amplification de la créatine kinase musculaire (MCK) chez le mammifère et au moins deux éléments d'amplification de la troponine 1 IRE (FIRE) chez le vertébré.

9. Elément de régulation spécifique aux muscles recombiné pour l'expression génique, comprenant au moins un élément d'amplification de la desmine (DES) chez le mammifère et un promoteur de la créatine kinase musculaire (MCK) chez le mammifère,
étant précisé que l'élément promoteur et l'élément d'amplification sont opérationnellement liés.

10. Elément de régulation selon l'une des revendications 1-9,
dans lequel
l'élément promoteur est un promoteur humain ou murin.

11. Elément de régulation selon l'une des revendications 1, 2 ou 9,
dans lequel
le promoteur MCK comporte le promoteur MCK humain selon SEQ ID NO :18.

12. Elément de régulation selon l'une des revendications 1-8,
dans lequel
le promoteur DES comprend le promoteur DES humain selon SEQ ID NO :19.

13. Elément de régulation selon l'une des revendications 1-9,
dans lequel
l'élément d'amplification est un élément d'amplification humain ou un élément d'amplification murin.

14. Elément de régulation selon l'une des revendications 1-6 ou 8,
dans lequel
l'élément de régulation comporte l'élément d'amplification MCK de la souris selon SEQ ID NO :20.

15. Elément de régulation selon l'une des revendications 3 ou 6-9,
dans lequel
l'élément de régulation comporte l'élément d'amplification DES humain selon SEQ ID NO :21.

16. Elément de régulation selon l'une des revendications 1, 3, 5, 6 ou 8
dans lequel
l'élément de régulation comprend un élément d'amplification FIRE qui est un élément d'amplification aviaire un élément d'amplification chez le mammifère ou un élément d'amplification humain.

17. Elément de régulation selon la revendication 16,
dans lequel
l'élément d"amplification FIRE comprend l'élément d'amplification de la troponine 1 chez la caille selon SEQ ID NO :22.

18. Elément de régulation selon l'une des revendications 1-7, 8, ou 9,
dans lequel
les éléments promoteurs et les éléments d'amplification sont dérivés de la même espèce ou sont dérivés d'espèces différentes.

19. Vecteur comprenant l'élément de régulation spécifique aux muscles recombiné pour l'expression génique qui est définie par l'une des revendication 1-18.

20. Vecteur selon la revendication 19,
choisi dans le groupe formé par un plasmide et un vecteur viral.

21. Vecteur selon la revendication 20,
dans lequel
le vecteur viral est dérivé d'un virus choisi dans le groupe constitué par un adénovirus, un virus adéno-associé et un rétrovirus.

22. Vecteur selon la revendication 21,
dans lequel
le rétrovirus est un lentivirus.

23. Utilisation du vecteur selon la revendication 19 pour la préparation d'un médicament afin d'exprimer un gène dans le muscle.

24. Cellule hôte transfectée comprenant le vecteur selon la revendication 19.

25. Cellule hôte transfectée selon la revendication 24,
dans laquelle
la cellule hôte est une cellule procaryotique ou une cellule de mammifère.

26. Cellule hôte transfectée selon la revendication 24,
dans laquelle
la cellule hôte est un myocyte.

27. Transgène recombiné pouvant être utilisé pour l'expression d'une séquence codante comprenant un promoteur constitutif fort et au moins un élément d'amplification spécifique aux muscles,
étant précisé que le promoteur constitutif fort est un promoteur de l'alpha actine squelettique (ASKA) de mammifère et que l'élément d'amplification spécifique aux muscles est choisi dans le groupe constitué par les éléments de régulation interne de la troponine 1 (FIRE) chez le mammifère, les éléments d'amplification de la créatine kinase musculaire (MCK) chez le mammifère et les éléments d'amplification de la desmine (DES) chez le mammifère.

28. Transgène recombiné pouvant être utilisé pour l'expression d'une séquence codante comprenant un promoteur constitutif fort et au moins un élément d'amplification spécifique aux muscles, le promoteur constitutif fort étant un promoteur de la troponine 1 (TNN12) chez le mammifère et l'élément d'amplification spécifique aux muscles étant choisi dans le groupe formé par les éléments d'amplification de la créatine kinase musculaire (MCK) chez le mammifère et les éléments d'amplification de la desmine (DES) chez le mammifère.

29. Transgène recombiné selon la revendication 27 ou 28,
dans lequel
l'élément promoteur est un promoteur humain ou murin.

30. Transgène recombiné selon la revendication 27 ou 28,
dans lequel
le promoteur est un promoteur tronqué dans lequel au moins un site de liaison de répresseurs de la transcription connue a été supprimé.

31. Vecteur comprenant le transgène selon la revendication 27 ou 28.

32. Vecteur selon la revendication 31,
choisi dans le groupe formé un plasmide et un vecteur viral.

33. Vecteur selon la revendication 32,
dans lequel
le vecteur viral est dérivé d'un virus choisi parmi un adénovirus, un virus adéno-associé et un rétrovirus.

34. Vecteur selon la revendication 33,
dans lequel
le rétrovirus est un lentivirus.

35. Utilisation du vecteur selon la revendication 31 pour la préparation d'un médicament pour l'expression d'un gène dans le muscle.

36. Cellule hôte transfectée comprenant le vecteur selon la revendication 31.

37. Cellule hôte transfectée selon la revendication 36,
dans laquelle
la cellule hôte est une cellule procaryotique ou une cellule de mammifère.

38. Cellule hôte transfectée selon la revendication 36,
dans laquelle
la cellule hôte est un myocyte.


### SEQUENCE LISTING

<110> Genzyme Corporation
   Souza, David
   Armentano, Donna
<120> Muscle-specific expression vectors
<130> 5059
<140> US 10/156,604
   <141> 2002-05-24
<150> US 60/293,304
   <151> 2001-05-24
<160> 26
<170> PatentIn version 3.2
<210> 1
   <211> 40
   <212> DNA
   <213> Artificial
<220>
   <223> Human DES promoter 5' primer
<400> 1
   gttgaattca catattgacc tctctttctt cctactcccc 40
<210> 2
   <211> 52
   <212> DNA
   <213> Artificial
<220>
   <223> Human DES promoter 3' primer
<400> 2
   ggtagatcta agccggtcct tgttcggcac tatttgtatc ccctcctgac at 52
<210> 3
   <211> 30
   <212> DNA
   <213> Artificial
<220>
   <223> Human DES enhancer 5' primer
<400> 3
   ggtactagtc ctgcccccac agctcctct 30
<210> 4
   <211> 43
   <212> DNA
   <213> Artificial
<220>
   <223> Human DES enhancer 3' primer
<400> 4
   ggtcgtacga attgctagca cagactttgt gtggctcctg ccc 43
<210> 5
   <211> 19
   <212> DNA
   <213> Artificial
<220>
   <223> MCK promoter s7 primer
<400> 5
   accctgaacc caggcatgc 19
<210> 6
   <211> 18
   <212> DNA
   <213> Artificial
<220>
   <223> MCK promoter 59 primer
<400> 6
   gcatgcctgg gttcaggt 18
<210> 7
   <211> 17
   <212> DNA
   <213> Artificial
<220>
   <223> MCK promoter s11 primer
<400> 7
   ccctgagttt gaatctc 17
<210> 8
   <211> 17
   <212> DNA
   <213> Artificial
<220>
   <223> MCK promoter s12 primer
<400> 8
   aaggggggct gtctgta 17
<210> 9
   <211> 33
   <212> DNA
   <213> Artificial
<220>
   <223> Mouse MCK enhancer 5' primer
<400> 9
   gggactagtc cactacggtc taggctgccc atg 33
<210> 10
   <211> 50
   <212> DNA
   <213> Artificial
<220>
   <223> Mouse MCK enhancer 3' primer
<400> 10
   gggcgtacga ttggtgctag catccaccag ggacagggtt atttttagag 50
<210> 11
   <211> 59
   <212> DNA
   <213> Artificial
<220>
   <223> FIRE 1
<400> 11
   gttactagtc ctggctgcgt ctgaggagac agctgcagct ccttgtgcag ctccccagc 59
<210> 12
   <211> 72
   <212> DNA
   <213> Artificial
<220>
   <223> FIRE 2
<400> 12
   gggtgggggg ggaaagtgct tctaaaaatg gctggggagc tgcacaagga gctgcagctg 60
   tctcctcaga cg 72
<210> 13
   <211> 60
   <212> DNA
   <213> Artificial
<220>
   <223> FIRE 3
<400> 13
   catttttaga agcactttcc ccccccaccc ccttgctctt cccagcaatg tgttgtgcct 60
<210> 14
   <211> 87
   <212> DNA
   <213> Artificial
<220>
   <223> FIRE 4
<400> 14
   ggtcgtacgg gtaagctagc caagctccct gaggaaacct tatcctggaa aatgtgcagg 60
   cacaacacat tgctgggaag agcaagg 87
<210> 15
   <211> 57
   <212> DNA
   <213> Artificial
<220>
   <223> FIRE 5
<400> 15
   gcacattttc caggataagg tttcctcagg gagcttggct agcttacccg tacgacc 57
<210> 16
   <211> 30
   <212> DNA
   <213> Artificial
<220>
   <223> Human ASKA promoter 5' primer
<400> 16
   ggtgaattca agtgggagtt tggggatctg 30
<210> 17
   <211> 30
   <212> DNA
   <213> Artificial
<220>
   <223> Human ASKA promoter 3' primer
<400> 17
   attaggatcc aagcgaggct tcacttggcg 30
<210> 18
   <211> 655
   <212> DNA
   <213> Homo sapiens
<400> 18
   cctgagtttg aatctctcca actcagccag cctcagtttc ccctccactc agtccctagg     60
   aggaaggggc gcccaagcgg gtttctgggg ttagactgcc ctccattgca attggtcctt    120
   ctcccggcct ctgcttcctc cagctcacag ggtatctgct cctcctggag ccacaccttg    180
   gttccccgag gtgccgctgg gactcgggta ggggtgaggg cccaggggcg acagggggag    240
   ccgagggcca caggaagggc tggtggctga aggagactca ggggccaggg gacggtggct    300
   tctacgtgct tgggacgttc ccagccaccg tcccatgttc ccggcggggg ccagctgtcc    360
   ccaccgccag cccaactcag cacttggtta gggtatcagc ttggtggggg cgtgagccca    420
   gccctggggc gctcagccca tacaaggcca tggggctggg cgcaaagcat gcctgggttc    480
   agggtgggta tggtgccgga gcagggaggt gagaggctca gctgccctcc agaactcctc    540
   cctggggaca acccctccca gccaatagca cagcctaggt ccccctatat aaggccacgg    600
   ctgctggccc ttcctttggg tcagtgtcac ctccaggata cagacagccc ccctt         655
<210> 19
   <211> 240
   <212> DNA
   <213> Homo sapiens
<400> 19
   ctgcagacat gcttgctgcc tgccctggcg aaggattggt aggcttgccg tcacaggacc     60
   cccgctggct gactcagggg cgcaggctct tgcgggggag ctggcctccc gcccccacgg    120
   ccacgggccc tttcctggca ggacagcggg atcttgcagc tgtcagggga ggggatgacg    180
   ggggactgat gtcaggaggg gatacaaata gtgccgaaca aggaccggat tagatctacc    240
<210> 20
   <211> 206
   <212> DNA
   <213> Mus musculus
<400> 20
   ccactacggg tctaggctgc ccatgtaagg aggcaaggcc tggggacacc cgagatgcct     60
   ggttataatt aacccagaca tgtggctgcc cccccccccc caacacctgc tgcctgagcc    120
   tcacccccac cccggtgcct gggtcttagg ctctgtacac catggaggag aagctcgctc    180
   taaaaataac cctgtccctg gtggat                                         206
<210> 21
   <211> 240
   <212> DNA
   <213> Homo sapiens
<400> 21
   cccccctgcc cccacagctc ctctcctgtg ccttgtttcc cagccatgcg ttctcctcta      60
   taaatacccg ctctggtatt tggggttggc agctgttgct gccagggaga tggttgggtt      120
   gacatgcggc tcctgacaaa acacaaaccc ctggtgtgtg tgggcgtggg tggtgtgagt      180
   agggggatga atcagggagg gggcggggga cccagggggc aggagccaca caaagtctgt      240
<210> 22
   <211> 148
   <212> DNA
   <213> Avian
<220>
   <223> FIRE enhancer
<400> 22
   cctggctgcg tctgaggaga cagctgcagc tccttgtgca gctccccagc catttttaga      60
   agcactttcc ccccccaccc ccttgctctt cccagcaatg tgttgtgcct gcacattttc      120
   caggataagg tttcctcagg gagcttgg                                         148
<210> 23
   <211> 516
   <212> DNA
   <213> Homo sapiens
<400> 23 ccaccgcagc ggacagcgcc aagtgaagcc tcgctt 516
<210> 24
   <211> 164
   <212> DNA
   <213> Homo sapiens
<400> 24
   gcggccaggc caggcggccg gacaggtggg gaggtctctg tggctctcca cgcccccatt 60
   ggtctgagga ggactctatg ccctttctga gcaggggccc agcctggggg aggccattta 120
   tacccctccc cctgggccca ccagcccaac tcgccgctgc cggc 164
<210> 25
   <211> 33
   <212> DNA
   <213> Artificial
<220>
   <223> Human TNI2 promoter 5' primer
<400> 25
   gttgaattcg cggccaggcc aggcggccgg aca 33
<210> 26
   <211> 30
   <212> DNA
   <213> Artificial
<220>
   <223> Human TNI2 promoter 3' primer
<400> 26
   gttggatcca ggccggcagc ggcgagttgg 30